# EUROPEAN PATENT SPECIFICATION

(11) **EP 3 308 751 B1**
(45) Date of publication and mention of the grant of the patent: **06.01.2021**
(21) Application number: 16799438.3
(22) Date of filing: 12.05.2016
(51) Int. Cl.: A61F 5/37, A61B 6/04

(54) **PELVIC DEVICE FOR TAKING X-RAYS**
BECKENVORRICHTUNG FÜR RÖNTGENSTRAHLEN
DISPOSITIF PELVIEN POUR PRISES DE RADIOGRAPHIES

(30) Priority: 26.05.2015 CO 15119622
(43) Date of publication of application: 18.04.2018
(73) Proprietor: Sarmiento Piñeros, Arturo Enrique, Bogotá 110221 (CO)
(72) Inventor: Sarmiento Piñeros, Arturo Enrique, Bogotá 110221 (CO)
(74) Representative: Dehns
(86) International application number: PCT/IB2016/052756
(87) International publication number: WO 2016/189410

(56) References cited:
- DE-A1-102012 012 382
- US-A- 3 606 885
- US-A- 3 933 154
- US-A- 4 576 151
- US-A- 5 814 001
- US-A1- 2014 251 350
- US-A1- 2014 251 350
- DATABASE WPI Week 201402, Derwent Publications Ltd., London, GB; AN 2013-X36623, XP055331712 & DE 10 2012 012382 A1 (SPOERER AG) 24 December 2013
- DATABASE WPI Week 198737, Derwent Publications Ltd., London, GB; AN 1987-264031, XP055331715 & WO 87 05207 A1 (RADL JOHANN JUN ET AL.) 11 September 1987

## Description

### FIELD OF THE INVENTION.

The present invention is related to a pelvic device for taking pelvic radiographs and for application in medicine, especially in children orthopedic.

### BACKGROUND OF THE INVENTION

The study of pelvic development diseases in children has been the subject of multiple researches highlighting pelvic radiograph as an easily available and low cost practical method for training its reading in health personnel. The limitation with these radiographs lies in exposure to radiation.

The diagnosis and treatment of developmental dysplasia of the hip has been a difficult problem for orthopedic surgeons, although there are several studies on the reliability of some of the measurement techniques that allow differentiating borderline patients, because parameters that may differ from one observer to another could be presented.

The first provision in the evaluation of pelvic radiographs in children is that the radiograph should have a good exposure quality and the position of the pelvis should not be rotated. This fact is highlighted, for example, by Boniforti who emphasized that radiographs should not be taken rotated. Based on this aspect, since 1975 authors such as Tönnis determine the norms in the radiographic measurements in his study: "With no rotation or inclination of the pelvis".

This author mentions the correct positioning for the pelvic radiograph, emphasizing the relevance of the reproducibility that each measurement should have. When the pelvis is rotated from its place, the angle of the hip that is rotated decreases and that of the opposite hip increases, this being progressive with the increase of the inclination. This rotation can be estimated by measuring the diameter of the obturator hole on each side and obtaining a neutral relation if the result equals 1 (arrow).

The degrees of inclination of the pelvis can be measured by evaluating the angle of the symphysis and ischium, drawing some lines from the most prominent point of the symphysis and another line with the highest point of the ischium, finding normal ranges in the population between 1.8 and 0.56.

However, in several studies reported in the literature, rotated radiographs or those considered to be taken with a deficient technique are considered as grounds for exclusion. In a cited example, the radiographic parameters proposed by Tönnis to better control the bias in the variability of the measurement that is complicated by the radiological technique is the high interobserver and intraobserver concordance with respect to the measurement of the acetabular index.

For this reason, a non-rotated and non-tilted radiograph is required, even if the study is repeated because this principle is of great importance. It should be taken into account that the radiation has some potential adverse effects that occur with the exposure in each radiographic shot, being an important factor to have a selectable image with the least possible amount of shots.

Given the importance of early detection and timely intervention in patients with developmental dysplasia of the hip, more attention has been given to the subtle cases of acetabular dysplasia, so accuracy in the measurement of radiographic indices is essential. However, there are potential sources of error in obtaining the measurement of the acetabular index as the rotations of the image. In this way, the goal is to keep the high concordance between measurements of pelvic radiographs for diagnosis, with high reproducibility of measures such as the acetabular index. Therefore, finding a device, which does not exist today in the state of the art, to be able to reduce to the maximum the possible biases resulting from the bad quality of the image, is the objective of the present pelvic anti-rotation device.

US 2014/0251350 discloses an immobilization device which includes a torso brace positioned around a subject's torso, a leg brace configured to be coupled around a respective leg, a torso brace joint assembly coupled to the torso brace and a respective connecting rod and includes a torso-brace ball and socket joint and a torso-brace locking mechanism.

DE 10 2012 012382 discloses a hip bracing orthosis with a stable base frame, which comprises a central longitudinal strut and an upper transverse running knee-spreader strut and a lower transverse running lower leg-spreader strut. The pivot angle of the joints is limited to a certain predetermined angle value by a pivot angle limiter.

### BRIEF SUMMARY OF THE INVENTION

The present invention relates to a pelvic device for taking pelvic radiographs which helps the correct positioning of the patient for the taking of the pelvis radiograph which leads to a more accurate diagnosis, thus avoiding, in addition to the inaccuracies in the diagnosis, a lower degree of radiation when the radiograph that the specialist requires for a correct diagnosis is obtained in a single shot.

Further objects and advantages of the present invention will become more apparent in the description of the figures, the detailed description of the invention and the claims.

### BRIEF DESCRIPTION OF THE DRAWINGS

The following drawings are intended to be explanatory and non-limiting of the present invention, in which:
Fig. 1 is a front view of a device not being part of the present invention.
Fig. 2 is a rear view of a device not being part of the present invention.
Fig. 3 is a left side view of a device not being part of the present invention.
Fig. 4 is an illustration of a part of the device of the invention with indication of some areas of the patient's body and parts of the device.
Fig. 5 is a three-dimensional representation of a pediatric patient placed on a device not being part of the present invention.
Fig. 5A, is a view of one embodiment of the device of the present invention seen from the back where rigid straps attached in a U-shape can be seen.
Fig. 5B, is a view of an embodiment of the device of the present invention seen from the front where rigid straps attached in a U-shape can be seen.
Figs 6 and 6A show another embodiment of the invention.

### DESCRIPTION OF THE PREFERRED EMBODIMENTS OF THE INVENTION

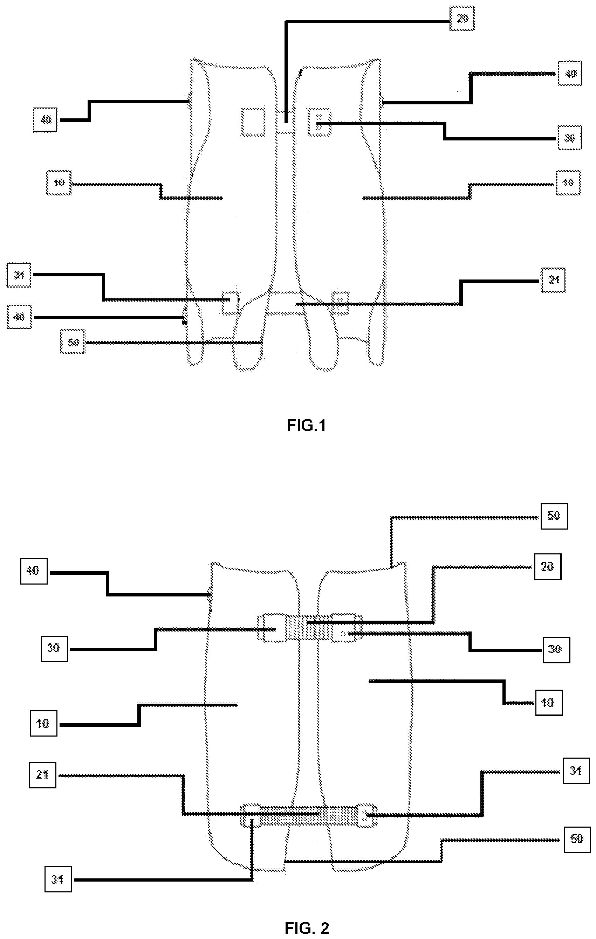

The invention is defined by independent claim 1. Further embodiments of the invention are defined by the dependent claims. The present disclosure i relates to a pelvic device for pelvic radiographs that allows the centering of the hip by blocking it in the proper position for the taking of radiographs in pediatric patients comprising one or more parts (10), in the case of the example two side parts in mirror image arrangement joined in the rear region with one or more adjustable rear joints or rigid straps (20, 21) can be seen as illustrated in figure 1. It is important to highlight that having two parts that are mirror images of each other, it is achieved that they are ergonomic and fit the pelvis and each of the thighs of a pediatric patient in a position of the hips at a specific angle of flexion and neutral rotation (figure 1, 2 and 3). These rigid straps can be one, two, three, four, five, six, seven or more depending on the design that is required.

Therefore, the present invention is a response to unsolved problems of the state of the art, since now it is achieved that the pelvic region of the patient, while said patient is lying on his/her back, keeps a flexion with a specific angle with the position of the floor and neutral rotation, which is decisive when taking a radiograph to diagnose a dysplasia in a correct way.

As shown in Figures 1, 2 and 3, a pelvic device not being part of the present invention is formed by two side parts (10) provided with rear links or rigid straps (20, 21) that join the two side parts and fix them.

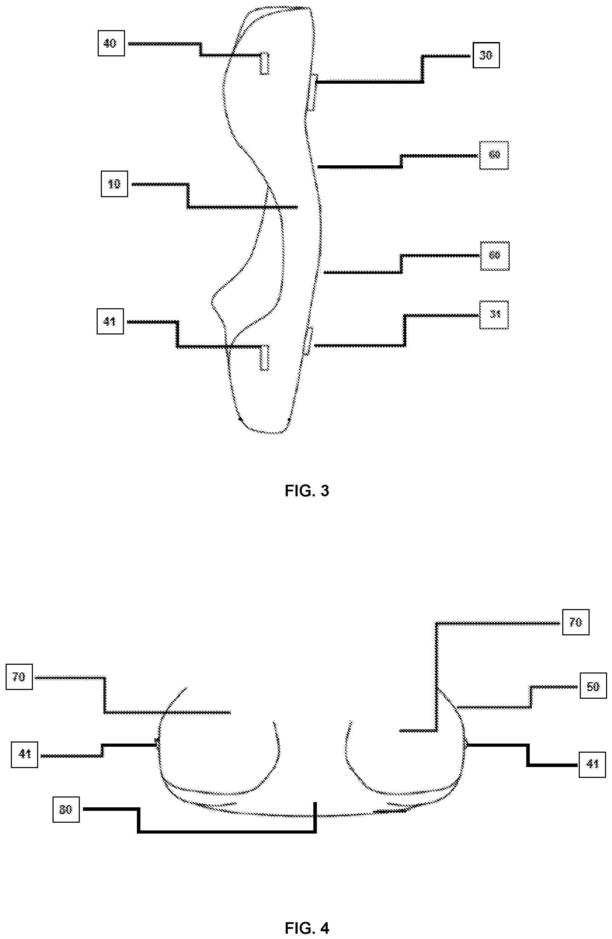

On the other hand, figure 3 shows, in addition to the side part (10), the slopes (60) that allow the patient to remain positioned for the taking of the radiograph, the device possesses rigid ears (30, 31) for the sliding of the rigid strap and allows to join and adjust the distance between the two side pieces and additionally optionally can have upper and lower eyelets (40, 41) through which one or more fastening straps (100, 110) slide. These belts can be made, for example, from Velcro.

Figure 4 shows the space for the thighs (70), the rigid lower eyelet (41), the gluteal position scheme (80) and the anatomical blunt ridges (50).

Said side parts (10), as already said, are located in a mirror image manner. As also mentioned, the device can have one or more rear joints or rigid straps (20, 21) that are located in the rear region of the device and allows to join and adjust the distance between the mirror image two side parts (10) to achieve the suitable disposition for each patient (figure 2). The material of said device parts is a radiolucent material such as polypropylene (Figure 3). In a preferred embodiment of the invention, the rigid straps (20, 21) can be toothed.

Said rigid toothed straps (20, 21) slide and fit within rigid ears (30, 31) located at each end of the straps that are fixed to each support in a mirror image manner and allow the adjustable and stable adjustment of the distance between each side parts (10). These side parts have a shape for the patient's thighs (figure 4).

The device in its side walls allows the arrangement of two toothed straps (20, 21) in its rear region, one upper and one lower (figure 2) that slide on rigid distal ears (30, 31) for adjustment, that is to say, they keep a certain distance and are fixed to the mirror image two side parts (10) and allow to adjust the distance between each of the side parts, facilitating the adjustment of the device to the different sizes of the patients.

In a preferred embodiment of the invention, the toothed straps are joined by means of a U-shaped handle (23) in such a way that these are not independent to give greater stability to the device.

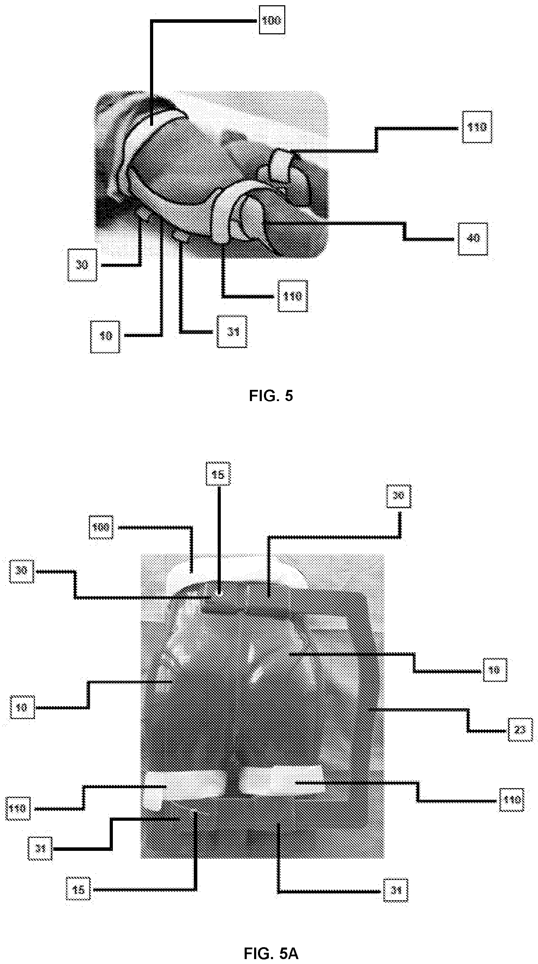

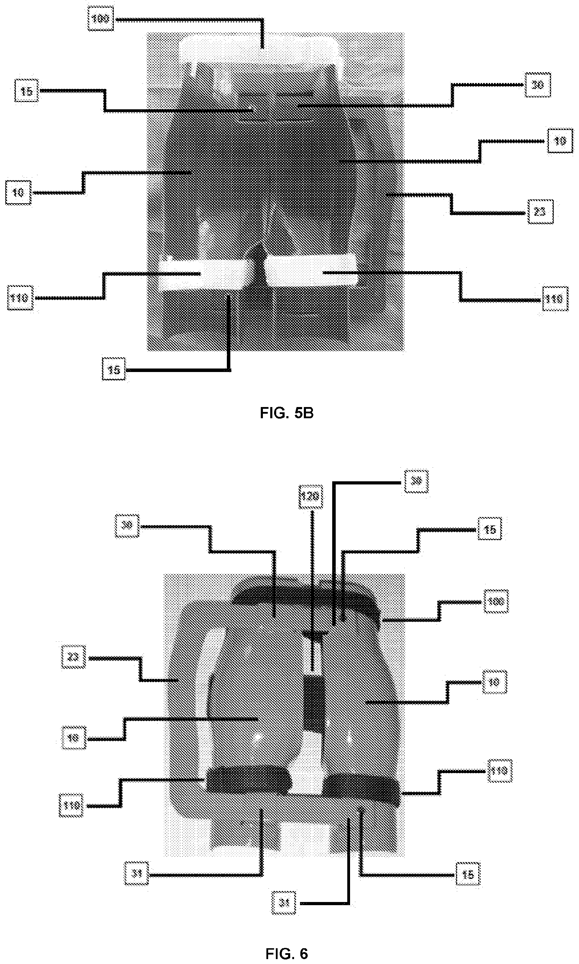

At each end of the device there are flexible straps (100, 110) that allow: the upper (100) to hold the hemiabdomen of the patient to the device and the lower two (110) to hold each patient's thigh, said straps slide by rigid eyelets (40, 41) that the device pose. As an example of the invention, said flexible bands or straps (100, 110) (Figure 5) can be made from Velcro and cotton padding, approximately 60 cm in length, the band (100) is unique and slides over the hemiabdomen of the patient to secure it to the upper region of the device and two straps (110), in the distal region, of approximately 30 cm each, allow each of the patient's thighs to be held from rear to front, they are located at a certain distance. These are not seen when taking the pelvic AP radiograph of the pediatric patient and are fastened or adjusted through three rigid eyelets (40).

In one embodiment of the present invention, the device has finishes on its ridges (50) that are blunt and a general anatomical shape of the gluteal region of a pediatric patient and that go to the front to allow the device to be attached to the patient in a safe and ergonomic way. The position scheme (80) is observed in figure 4 as well as the room (70) for the thighs of the pediatric patient.

For practical purposes, the system is complemented with presentations in two sizes, expanding the possibility of sizes of pediatric patients who can use the device.

The device of the present invention generates stable positioning, is clean, low allergenic, speeds up and standardizes the taking of pelvic AP radiographs in pediatric patients who cannot maintain a suitable position for taking the radiograph.

The device of the present invention can be manufactured based on radiolucent materials, for example, in low density polypropylene where the finishes can be of polypropylene color or elaborated in the desired shade.

In Figure 5, the way the device used in which the patient is seen and the manner in which the side parts (10) are opened and closed by the rear joints or rigid straps (20, 21) and the straps (100, 110) that are used to immobilize the patient is illustrated.

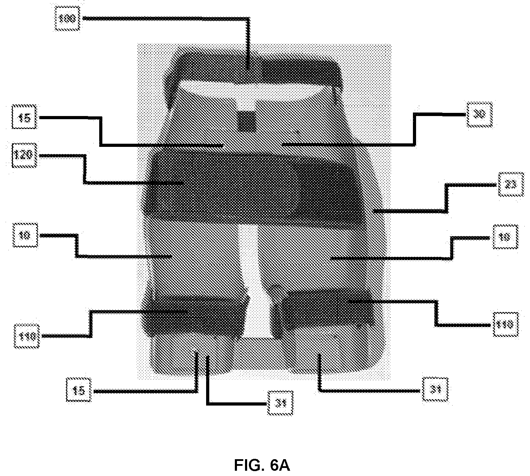

Finally, in an embodiment of the invention (Figures 6 and 6A), the present device fixes the rigid straps (20, 21) joined with the U-shaped handle (23) with a fixing element (15) (for example , rivets, screw and nut, etc.) to one of the side parts (10) leaving the other accommodating to adjust any patient and where the rigid straps or rear joints (20, 21) are not toothed but smooth. Additionally, this method not only has two types of flexible straps (100, 110) that fix the abdomen and thighs of the pediatric patient, but also has a third type of flexible strap (120), which fixes the pelvis of said patient, this in order to give even greater stability and further limit the possibilities of an inadequate radiograph due to patient movements, that is, there may be one or more flexible straps or fixation elements to immobilize the pediatric patient.

As an example of a device of the invention, it can be manufactured with the following elements:
1. Two side pieces.
   a. Made in radiolucent low-density polypropylene with finishes in the color of polypropylene or made in the desired shade.
2. Two rigid straps: a. made in radiolucent low-density polypropylene.
3. Three flexible straps.
   a. One cotton-padded velcro upper strap 60 cm long and 2 cm wide to secure the patient's hemiabdomen to the device.
   b. Two cotton-padded velcro lower strap 30 cm long each to ensure each of the patient's thighs to the device.
4. Low density polypropylene 4 mm thick Lot 032015

The advantages of the anti-rotational pelvic device are:
1. Decrease the repetitive taking of the radiographs by inadequate technique
2. Reduce the measurement bias of the radiograph by standardizing the patient's position at the time of the study.
3. Decrease the number of people receiving radiation together with the pediatric patient.
4. Safe place that prevents the child from falling or hurting when falling from a smooth and uncomfortable surface.
5. Increase the probability of a better radiograph shot with a single shot.
6. Decrease the time between pediatric patients who require pelvic radiograph.
7. Economic method of support for the pediatric patient.
8. Durable support method for the pediatric patient.
9. Safe and adjustable method for holding the pediatric patient.
10. Radiolucent device that allows adequate patient safety and does not interfere with the image obtained.
11. Optimize the position of the pediatric patient on the X-ray table to decrease the rotation of the pelvis to zero.
12. Decrease the time between pediatric patients who require pelvic radiograph.

## Claims

1. A pelvic device for patient positioning during radiographs, the pelvic device comprising two side parts (10), with a shape configured to receive a patient's thighs, the two side parts (10) provided with two or more rigid straps (20, 21) that join the two side parts and fix them, and one or more immobilization elements (100, 110, 120), wherein the side parts (10) are mirror image arranged, **characterised in that** the rigid straps (20, 21) are joined by a U-shaped handle (23), have rigid ears (30, 31 for their adjustment and are adjustable.

2. The pelvic device of claim 1, wherein said rigid straps (20, 21) are smooth or toothed.

3. The pelvic device of claim 1 or 2, **characterized in that** the immobilization elements (100, 110, 120) are flexible and padded.

4. The pelvic device of claim 3, **characterized in that** the immobilization elements (100, 110) are flexible straps which have rigid eyelets (40, 41) for adjustment.

5. The pelvic device of claim 1, **characterized in that** its ridges (50) are blunt.

6. The pelvic device of any of the preceding claims **characterized in that** one of the immobilization elements (100) is configured to fix the patient's abdomen, other immobilization elements (110) are configured to immobilize the legs and still others (120) are configured to fix the patient's hip.

7. The pelvic device of any of the preceding claims which is made of a radiolucent material.

## Patentansprüche

1. Beckenvorrichtung zur Patientenpositionierung während Röntgenaufnahmen,
wobei die Beckenvorrichtung zwei Seitenteile (10) umfasst, mit einer Form, die konfiguriert ist, um die Oberschenkel eines Patienten aufzunehmen, wobei die zwei Seitenteile (10) mit zwei oder mehr starren Riemen (20, 21) bereitgestellt sind, die die zwei Seitenteile verbinden und sie fixieren, und ein oder mehr Immobilisierungselemente (100, 110, 120), wobei die Seitenteile (10) spiegelbildlich angeordnet sind, **dadurch gekennzeichnet, dass** die starren Riemen (20, 21) durch einen U-förmigen Griff (23) verbunden sind, starre Ohren (30, 31) für ihre Einstellung aufweisen und einstellbar sind.

2. Beckenvorrichtung nach Anspruch 1, wobei die starren Gurte (20, 21) glatt oder gezahnt sind.

3. Beckenvorrichtung nach Anspruch 1 oder 2, **dadurch gekennzeichnet, dass** die Immobilisierungselemente (100, 110, 120) flexibel und gepolstert sind.

4. Beckenvorrichtung nach Anspruch 3, **dadurch gekennzeichnet, dass** die Immobilisierungselemente (100, 110) flexible Gurte sind, die starre Ösen (40, 41) zur Einstellung aufweisen.

5. Beckenvorrichtung nach Anspruch 1, **dadurch gekennzeichnet, dass** ihre Rippen (50) stumpf sind.

6. Beckenvorrichtung nach einem der vorstehenden Ansprüche, **dadurch gekennzeichnet, dass** eines der Immobilisierungselemente (100) so konfiguriert ist, dass es den Bauch des Patienten fixiert, andere Immobilisierungselemente (110) so konfiguriert sind, dass sie die Beine immobilisieren und wieder andere (120) so konfiguriert sind, dass sie die Hüfte des Patienten immobilisieren.

7. Beckenvorrichtung nach einem der vorstehenden Ansprüche, die aus einem strahlendurchlässigen Material hergestellt ist.

## Revendications

1. Dispositif pelvien pour le positionnement de patient lors de radiographies,
le dispositif pelvien comprenant deux parties latérales (10), avec une forme configurée pour recevoir les cuisses d'un patient, les deux parties latérales (10) étant dotées de deux ou plusieurs sangles rigides (20, 21) qui rejoignent les deux parties latérales et les maintiennent, et un ou plusieurs éléments d'immobilisation (100, 110, 120),
dans lequel les parties latérales (10) sont agencées symétriquement,
**caractérisé en ce que** les sangles rigides (20, 21) sont jointes par une poignée en U (23), ont des oreilles rigides (30, 31) pour leur réglage et sont réglables.

2. Dispositif pelvien selon la revendication 1, dans lequel lesdites sangles rigides (20, 21) sont lisses ou grenues.

3. Dispositif pelvien selon la revendication 1 ou 2, **caractérisé en ce que** les éléments d'immobilisation (100, 110, 120) sont souples et rembourrés.

4. Dispositif pelvien selon la revendication 3, **caractérisé en ce que** les éléments d'immobilisation (100, 110) sont des sangles souples qui ont des œillets rigides (40, 41) destinés au réglage.

5. Dispositif pelvien selon la revendication 1, **caractérisé en ce que** ses arêtes (50) sont émoussées.

6. Dispositif pelvien selon l'une quelconque des revendications précédentes **caractérisé en ce que** l'un des éléments d'immobilisation (100) est configuré pour maintenir l'abdomen d'un patient, d'autres éléments d'immobilisation (110) sont configurés pour immobiliser les jambes et d'autres encore (120) sont configurés pour maintenir la hanche d'un patient.

7. Dispositif pelvien selon l'une quelconque des revendications précédentes qui est composé d'un matériau perméable aux rayons X.
